(19)

**Europäisches Patentamt**

**European Patent Office**

**Office européen des brevets**

(11) **EP 4 506 689 A1**

# EUROPEAN PATENT APPLICATION

(12)

(43) Date of publication:
**12.02.2025  Bulletin 2025/07**

(21) Application number: **24189039.1**

(22) Date of filing: **17.07.2024**

(51) International Patent Classification (IPC):
**G01N 33/44** *(2006.01)*

(52) Cooperative Patent Classification (CPC):
**G01N 33/445; G01N 33/44; G01N 33/442**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.08.2023   JP 2023127055**
**26.01.2024   JP 2024010003**

(71) Applicant: **Toyo Tire Corporation**
**Itami-shi, Hyogo 664-0847 (JP)**

(72) Inventors:
• **KOBIKI, Yohsuke**
**ITAMI-SHI, HYOGO, 664-0847 (JP)**
• **KUBO, Haruka**
**ITAMI-SHI, HYOGO, 664-0847 (JP)**
• **KIMURA, Takuya**
**ITAMI-SHI, HYOGO, 664-0847 (JP)**

(74) Representative: **Cabinet Beau de Loménie**
**158, rue de l'Université**
**75340 Paris Cedex 07 (FR)**

(54) **METHOD FOR QUANTIFYING COMPONENT IN RUBBER COMPOSITION**

(57)     A method for quantifying a component in a rubber composition, the method comprising at least: an extraction step in which, from the rubber composition, an extraction liquid containing an extract is obtained using an organic solvent; a concentration step in which the organic solvent is distilled off from the extraction liquid to obtain a concentrate of the extract; a thermal analysis step in which the concentrate is subjected to thermogravimetric analysis to obtain a thermogravimetric curve; and a quantification step in which a weight reduction ratio corresponding to a component to be quantified is read from the thermogravimetric curve. It is preferred that the component to be quantified is a non-volatile component. It is preferred that the non-volatile component is a hydrocarbon resin.

**EP 4 506 689 A1**

**Description**

Field of the Invention

The present invention relates to a method for quantifying a component in a rubber composition.

Description of the Related Art

Rubber compositions are used for many rubber products such as pneumatic tires. In order to understand and improve the performance of a rubber product, a technique to quantify a component contained in the rubber product is important.

Patent Document 1 mentioned below discloses a method for quantifying a component in a rubber composition, in which a solvent extract obtained by drying a solvent extraction liquid of a rubber composition containing a reference sample is analyzed by a pyrolysis gas chromatograph mass spectrometer to obtain a chromatogram, and a component kneaded with a rubber component is quantified as a quantification target component on the basis of a ratio between the peak area of a peak derived from the reference sample and the peak area of a peak derived from the quantification target component in the chromatogram.

Patent Document 2 mentioned below discloses a method for individually determining contents of respective resin components in a paste containing a plurality of resin components and an inorganic powder, the method including: a dissolution step in which the paste and an organic solvent are mixed to dissolve the resin components; a separation step in which the inorganic powder is separated from a solution obtained in the dissolution step to form a resin solution containing the resin components; a measurement step in which the resin solution is measured by pyrolysis gas chromatography to determine peak area values of pyrolysis products intrinsic to the respective resin components generated by pyrolysis of the respective resin components; a quantitative determination step in which contents of the respective resin components in the paste are calculated from the peak area values determined in the measurement step on the basis of a calibration curve representing a correlation between the contents and the peak area values of the intrinsic pyrolysis products; and between the separation step and the measurement step, a drying step in which the resin solution is dried by heating at a temperature equal to or higher than a boiling point of the organic solvent and equal to or lower than pyrolysis temperatures of the resin components to vaporize the organic solvent and form a dried product containing the resin components, wherein in the measurement step, the dried product is measured by pyrolysis gas chromatography.

Prior Art Documents

Patent Documents

   Patent Document 1: JP-B2-6665629
   Patent Document 2: JP-B2-6950494

SUMMARY OF THE INVENTION

The present inventors have intensively studied and as a result have found that the above-described conventional quantification methods have a limitation in components to be quantified because it is necessary to previously know information about components to be quantified (chemical structures and behaviors during control measurement) to accurately perform quantification. Therefore, the above-described conventional quantification methods have been found to still have room for improvement in versatility of components to be quantified.

In light of such circumstances, it is an object of the present invention to provide a method for quantifying a component in a rubber composition which is excellent in versatility of components to be quantified while achieving quantitativeness.

The above object can be achieved by the following configurations. Specifically, the present invention relates to a method for quantifying a component in a rubber composition (1), the method including at least: an extraction step in which, from the rubber composition, an extraction liquid containing an extract is obtained using an organic solvent; a concentration step in which the organic solvent is distilled off from the extraction liquid to obtain a concentrate of the extract; a thermal analysis step in which the concentrate is subjected to thermogravimetric analysis to obtain a thermogravimetric curve; and a quantification step in which a weight reduction ratio corresponding to a component to be quantified is read from the thermogravimetric curve.

The method for quantifying a component in a rubber composition (1) is preferably a method for quantifying a component in a rubber composition (2), wherein the component to be quantified is a non-volatile component.

The method for quantifying a component in a rubber composition (2) is preferably a method for quantifying a component in a rubber composition (3), wherein the non-volatile component is a hydrocarbon resin.

Any one of the methods for quantifying a component in a rubber composition (1) to (3) is preferably a method for quantifying a component in a rubber composition (4), wherein when, in the concentration step, a ratio of extraction amount of the concentrate as determined by taking a total amount of the rubber composition as 100 mass% is defined as $\alpha$ (mass%) and when after the thermal analysis step, a component whose weight is reduced in a high-temperature region is determined by further differentiating the thermogravimetric curve and, in the quantification step, a weight reduction ratio of the component in the high-temperature region is defined as $\beta$ (mass%), a content $\gamma$ (mass%) of the component in the rubber composition is calculated on the basis of the following formula (2):

$$\gamma = \alpha \times \beta/100 \quad (2).$$

Any one of the methods for quantifying a component in a rubber composition (1) to (4) is preferably a method for quantifying a component in a rubber composition (5), wherein the thermal analysis step is performed in an inert gas atmosphere.

The method for quantifying a component in a rubber composition (4) or (5) is preferably a method for quantifying a component in a rubber composition (6), wherein a differential curve of the thermogravimetric curve corresponding to the component whose weight is reduced in the high-temperature region has a peak top temperature of 250°C or higher.

Particularly, a rubber composition for use in pneumatic tires often contains a non-volatile component such as a hydrocarbon resin, and therefore a technique to quantify a component contained in a rubber product, especially a non-volatile component such as a hydrocarbon resin is important to understand and improve the performance of the rubber product. In this regard, there is a technique in which a non-volatile component such as a hydrocarbon resin is extracted by solvent extraction and the content thereof is determined on the basis of the amount of an extract. However, there is a problem that the amount of a target component cannot accurately be determined by such a technique because the extract contains not only the target component but also many other components such as an antiaging agent and oil.

On the other hand, in the present invention, an extraction step is performed using an organic solvent to obtain, from a rubber composition, an extraction liquid containing an extract, and then a concentrate of the extract is obtained by distilling off the organic solvent from the extraction liquid (concentration step). The quantification method according to the present invention further includes at least a thermal analysis step in which the concentrate is subjected to thermogravimetric analysis to obtain a thermogravimetric curve and a quantification step in which a weight reduction ratio corresponding to a component to be quantified is read from the thermogravimetric curve. This makes it possible to more accurately quantify a target component as compared to the conventional technique.

Particularly, in the present invention, a target component can even more accurately be quantified in a case where when, in the concentration step, a ratio of extraction amount of the concentrate as determined by taking a total amount of the rubber composition as 100 mass% is defined as $\alpha$ (mass%) and when after the thermal analysis step, a component whose weight is reduced in a high-temperature region is determined by further differentiating the thermogravimetric curve and, in the quantification step, a weight reduction ratio of the component in the high-temperature region is defined as $\beta$ (mass%), a content $\gamma$ (mass%) of the component in the rubber composition is calculated on the basis of the following formula (2):

$$\gamma = \alpha \times \beta/100 \quad (2).$$

It should be noted that in a differential curve of the thermogravimetric curve, a peak top temperature of a peak corresponding to the component to be quantified is preferably 250°C or higher. In this case, the component to be quantified can more accurately be quantified.

The present invention is superior in versatility of components to be quantified to the conventional technique, and is therefore preferred when a non-volatile component, especially a hydrocarbon resin is quantified as a target component.

It should be noted that the present invention is preferred when a component of a rubber composition after vulcanization (vulcanized rubber) as a rubber composition is quantified because quantitativeness is enhanced when a non-volatile component, especially a hydrocarbon resin is quantified.

BRIEF DESCRIPTION OF THE DRAWINGS

Fig. 1 is a diagram showing an example of a thermogravimetric analysis result (thermogravimetric curve) of a concentrate of an extract obtained by subjecting a rubber composition to extraction using chloroform as an organic solvent; and

Fig. 2 is a diagram showing a relationship between a quantification result (mass%) obtained by the quantification method according to the present invention or a conventional extraction method and an actual content (mass%).

DETAILED DESCRIPTION OF THE PREFERRED EMBODIMENTS

The present invention relates to a method for quantifying a component in a rubber composition, the method including at least: (i) an extraction step in which, from the rubber composition, an extraction liquid containing an extract is obtained using an organic solvent; (ii) a concentration step in which the organic solvent is distilled off from the extraction liquid to obtain a concentrate of the extract; (iii) a thermal analysis step in which the concentrate is subjected to thermogravimetric analysis to obtain a thermogravimetric curve; and (iv) a quantification step in which a weight reduction ratio corresponding to a component to be quantified is read from the thermogravimetric curve. Each of the steps will be described below.

(i) Extraction step

In the extraction step, an extraction liquid containing an extract is obtained using an organic solvent from a rubber composition, preferably a rubber composition after vulcanization. The organic solvent is a solvent usually used for extraction from a rubber composition, and specific examples thereof include chloroform, 1,2-dichloroethane, toluene, acetone, tetrahydrofuran, methanol, ethanol, 2-propanol, and ethyl acetate. Among them, chloroform is preferably used in the present invention. Conditions for obtaining, using an organic solvent, an extraction liquid containing an extract from a rubber composition are not limited, but from the viewpoint of more simply performing the subsequent concentration step and the viewpoint of further enhancing quantification accuracy, the extraction step is preferably performed at a ratio of 0.5 to 3.0 g of the rubber composition per 50 to 200 mL of the organic solvent. Further, a method for subjecting a rubber composition to extraction using an organic solvent is not limited and may be, for example, Soxhlet extraction. The extraction time for Soxhlet extraction is, for example, about 4 to 8 hours.

(ii) Concentration step

After the extraction step, the organic solvent is distilled off from the extraction liquid to obtain a concentrate of the extract (concentration step). A method for concentrating the extraction liquid is not limited and may be, for example, a method in which the organic solvent is distilled off by drying the extraction liquid under reduced pressure.

In the conventional technique based on an extraction method, the amount of a component extracted through the extraction step and the concentration step (the extraction amount of the concentrate) is regarded as an actual content in the rubber composition. On the other hand, in the method for quantifying a component in a rubber composition according to the present invention, on the basis of information obtained in the thermal analysis step and the quantification step described below, a content close to an actual content can be derived more accurately than when the amount of a component extracted is regarded as an actual content. The thermal analysis step and the quantification step will be described below.

(iii) Thermal analysis step

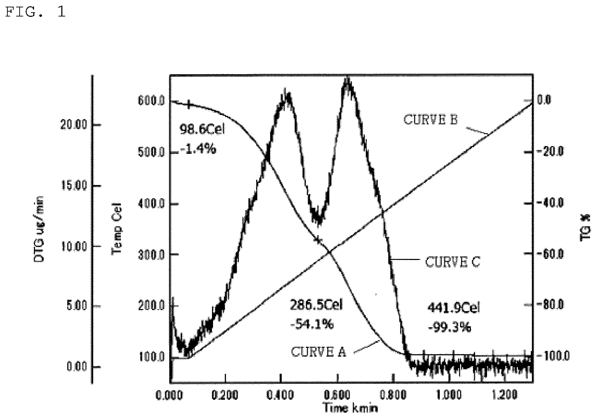

In the thermal analysis step, the concentrate is subjected to thermogravimetric analysis (hereinafter also simply referred to as "TGA") to obtain a thermogravimetric curve. Fig. 1 shows an example of a thermogravimetric analysis result (thermogravimetric curve) of a concentrate of an extract obtained by subjecting a rubber composition to extraction using chloroform as an organic solvent. In Fig. 1, the horizontal axis represents an elapsed time during heating (Time (kmin) The right vertical axis represents a thermal weight loss (TG (%)). A curve A is a thermogravimetric curve in which when the elapsed time during heating (Time) is 0.000, the thermal weight loss (TG) is 0.0, the weight loss gradually increases as heating continues, and the thermal weight loss (TG) is almost constant (-99.3%) after an elapsed time during heating (Time) of 0.800. The inner left vertical axis represents a heating temperature (Temp(°C)). A curve B is a heating curve in which when the elapsed time during heating (Time) is 0.000, the heating temperature is about 100°C, and the heating temperature finally increases to 600°C. The outer left vertical axis represents a thermal weight loss per minute (DTG ($\mu$g/min)). A curve C is a differential curve of the thermogravimetric curve (curve A) in which two maximum peaks appear when the elapsed time during heating (Time) is 0.400 to 0.500 and when the elapsed time during heating (Time) is 0.600 to 0.700.

In the present invention, from the viewpoint of preventing oxidation of the component to be quantified to more accurately measure a thermal weight loss, the thermal analysis step is preferably performed in an inert gas atmosphere.

(iv) Quantification step

In the quantification step, a weight reduction ratio corresponding to a component to be quantified is read from the thermogravimetric curve obtained in the thermal analysis step. In the present invention, a weight reduction ratio (β (mass%)) of the component in a high-temperature region is preferably read in the quantification step. Hereinbelow, a description will be made with reference to a case where the component to be quantified in the present invention is a non-volatile component whose peak top temperature in a differential curve of the thermogravimetric curve is 250°C or higher. A differential curve of the thermogravimetric curve obtained in the thermal analysis step has two maximum peaks, and a high-temperature-side maximum peak of them indicates that the weight loss of a non-volatile component increases . On the other hand, a low-temperature-side maximum peak indicates that the weight loss of another low-temperature volatile component, for example, an organic compounding agent in a vulcanized rubber increases. That is, in the case of the example shown by this diagram, in consideration of overlapping between the two maximum peaks, the ratio of weight loss from 286.5°C to 441.9°C corresponds to the weight reduction ratio (β (mass%)) of the non-volatile component in the high-temperature region.

It should be noted that the method for quantifying a component in a rubber composition, especially a rubber composition after vulcanization which includes a thermal analysis step in which a concentrate is subjected to thermogravimetric analysis to obtain a thermogravimetric curve has first been found by the present inventors as far as the present inventors know.

Particularly, in the present invention, when in the concentration step, a ratio of extraction amount of the concentrate as determined by taking a total amount of the rubber composition as 100 mass% is defined as α (mass%) and when after the thermal analysis step, a component whose weight is reduced in a high-temperature region is determined by further differentiating the thermogravimetric curve and, in the quantification step, a weight reduction ratio of the component in the high-temperature region is defined as β (mass%), a content γ (mass%) of the component in the rubber composition is preferably calculated on the basis of the following formula (2) :

$$\gamma = \alpha \times \beta/100 \quad (2).$$

In this case, quantitativeness of a non-volatile component is excellent.

In the present invention, the component to be quantified, which is contained in a rubber composition, preferably a rubber composition after vulcanization, is preferably a component whose peak top temperature in a differential curve of the thermogravimetric curve is 250°C or higher. When the component to be quantified is a component whose peak top temperature in a differential curve of the thermogravimetric curve is 250°C or higher, the component can more accurately be quantified. Such a component can be said as a non-volatile component, and the non-volatile component is preferably a hydrocarbon resin.

Examples

The present invention will more specifically be described below with reference to examples.

First, Formulation No. 1 to Formulation No. 7 were prepared as rubber compositions according to formulations shown in Table 1. Information about components shown in Table 1 is as follows.

- SBR; trade name "HPR840", manufactured by ENEOS Materials Corporation
- BR; trade name "BR730", manufactured by ENEOS Materials Corporation
- CB; trade name "DIABLACK N339", manufactured by Mitsubishi Chemical Corporation
- Silica; trade name "Nipsil AQ", manufactured by Nippon Silica Industries Corporation
- Cp agent; trade name "NXT SILANE", manufactured by Momentive Performance Materials
- Processing aid; trade name "AFLUX 16", manufactured by LANXESS
- Wax; trade name "OZOACE-2701", manufactured by NIPPON SEIRO CO., LTD.
- Stearic acid; trade name "Stearic Acid N-50", manufactured by NOF CORPORATION
- Zinc white; trade name "Zinc oxide grade 2", manufactured by SAKAI CHEMICAL INDUSTRY CO., LTD.
- 6PPD; trade name "NOCRAC 6C", manufactured by OUCHI SHINKO CHEMICAL INDUSTRIAL CO., LTD.
- TMDQ; trade name "ANTIOXIDANT TMQ", manufactured by Kemai Chemical
- Non-volatile component (hydrocarbon resin)

    (i) C9-C5 resin (peak top temperature of differential curve of weight reduction during TGA measurement (367°C)); trade name "Petrotack 90", manufactured by Tosoh Corporation
    (ii) Terpene resin (peak top temperature of differential curve of weight reduction during TGA measurement (360°C)); trade name "Sylvatraxx4150", manufactured by Kraton Chemical
(iii) Styrene resin (peak top temperature of differential curve of weight reduction during TGA measurement (368°C)) ; trade name "Sylvatraxx4401", manufactured by Kraton Chemical

· DPG; trade name "SOXINOL D-G", manufactured by SUMITOMO CHEMICAL COMPANY, LIMITED
· CBS; trade name "NOCCELER CZ-G", manufactured by OUCHI SHINKO CHEMICAL INDUSTRIAL CO., LTD.
· Sulfur; trade name "OIL TREATED SULFUR POWDER 150 mesh" , manufactured by Tsurumi Chemical Industry Co., ltd.

[Table 1]

| | | Formulation No. 1 | Formulation No. 2 | Formulation No. 3 | Formulation No. 4 | Formulation No. 5 | Formulation No. 6 | Formulation No. 7 |
|---|---|---|---|---|---|---|---|---|
| (Formulation) | | | | | | | | |
| SBR | | 30 | 30 | 30 | 30 | 30 | 30 | 30 |
| BR | | 70 | 70 | 70 | 70 | 70 | 70 | 70 |
| CB | | 5 | 5 | 5 | 5 | 5 | 5 | 5 |
| Silica | | 105 | 105 | 105 | 60 | 65 | 70 | 80 |
| Cp agent | | 8.4 | 8.4 | 8.4 | 4.8 | 5.2 | 5.6 | 6.4 |
| Processing aid | | 4.5 | 4.5 | 4.5 | 4.5 | 4.5 | 4.5 | 4.5 |
| Wax | | 2.1 | 2.1 | 2.1 | 2.1 | 2.1 | 2.1 | 2.1 |
| Stearic acid | | 2 | 2 | 2 | 2 | 2 | 2 | 2 |
| Zinc white | | 2 | 2 | 2 | 2 | 2 | 2 | 2 |
| 6PPD | | 2 | 2 | 2 | 2 | 2 | 2 | 2 |
| TMDQ | | 0.7 | 0.7 | 0.7 | 0.7 | 0.7 | 0.7 | 0.7 |
| Non-volatile component (Hydrocarbon resin) | C9-C5 resin | | 44 | | | 5 | 10 | 20 |
| | Terpene resin | 44 | | | | | | |
| | Styrene resin | | | 44 | | | | |
| DPG | | 2.3 | 2.3 | 2.3 | 2.3 | 2.3 | 2.3 | 2.3 |
| CBS | | 2.1 | 2.1 | 2.1 | 2.1 | 2.1 | 2.1 | 2.1 |
| Sulfur | | 1.7 | 1.7 | 1.7 | 1.7 | 1.7 | 1.7 | 1.7 |
| (Quantification results) | | | | | | | | |
| Quantification result of content (mass%) of hydrocarbon resin obtained on the basis of quantification method based on extraction amount ("EXTRACTION METHOD" in Fig. 2) = $\alpha$ (%) | | 21.2 | 20.9 | 21.1 | 7.2 | 8.5 | 11.8 | 15.5 |
| Weight reduction (mass%) of hydrocarbon resin in high-temperature region = $\beta$ (%) | | 69.8 | 68.0 | 66 . 6 | 3.7 | 37.0 | 45.3 | 60.1 |

(continued)

| (Quantification results) | | | | | | | |
|---|---|---|---|---|---|---|---|
| Quantification result of content of hydrocarbon resin obtained on the basis of quantification method according to present invention ("PRESENT INVENTION" in Fig. 2) = $\gamma = \alpha \times \beta / 100$ | 14.8 | 14.2 | 14.1 | 0.3 | 3.2 | 5.4 | 9.3 |
| Actual content (mass%) of hydrocarbon resin | 15.6 | 15.6 | 15.6 | 0.0 | 2.5 | 4.8 | 8.7 |

EP 4 506 689 A1

In Table 1, "Actual content (mass%) of hydrocarbon resin" refers to the total content (mass%) of "C9-C5 resin", "terpene resin", and "styrene resin" as a non-volatile component (hydrocarbon resin) when the total amount of the rubber composition after vulcanization is taken as 100 mass%.

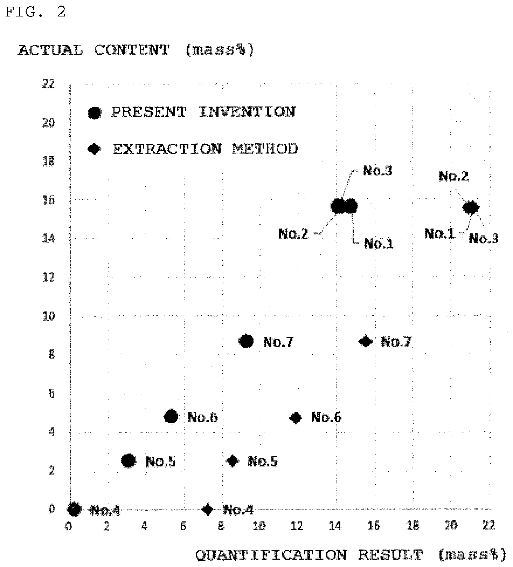

First, the content of a non-volatile component (hydrocarbon resin) in each of Formulation No. 1 to Formulation No. 7 was determined on the basis of a conventional quantification method based on extraction amount ("EXTRACTION METHOD" in Fig. 2). Specifically, an extraction liquid containing an extract was obtained using chloroform (organic solvent) from each of the rubber compositions after vulcanization of Formulation No. 1 to Formulation No. 7 (extraction step), and chloroform was distilled off from the extraction liquid to obtain a concentrate of the extract (concentration step). The content of a non-volatile component (hydrocarbon resin) determined on the basis of the amount (mass%) of concentrate of the extract is shown in the column "Quantification result of content (mass%) of hydrocarbon resin obtained on the basis of quantification method based on extraction amount ("EXTRACTION METHOD" in Fig. 2) " in Table 1.

Then, the content of a non-volatile component (hydrocarbon resin) in each of Formulation No. 1 to Formulation No. 7 was determined on the basis of the quantification method according to the present invention ("PRESENT INVENTION" in Fig. 2). Specifically, an extraction liquid containing an extract was obtained using chloroform (organic solvent) from each of the rubber compositions after vulcanization of Formulation No. 1 to Formulation No. 7 (extraction step), and chloroform was distilled off from the extraction liquid to obtain a concentrate of the extract (concentration step). The content of a non-volatile component (hydrocarbon resin) (ratio of extraction amount of the concentrate) determined on the basis of the amount of concentrate of the extract was defined as $\alpha$ (mass%). It should be noted that the $\alpha$ (mass%) is the same as the quantification result of content (mass%) of the hydrocarbon resin obtained on the basis of the extraction method. Then, the concentrate was subjected to thermogravimetric analysis to obtain a thermogravimetric curve (thermal analysis step), and a weight reduction ratio corresponding to a component to be quantified was read from the thermogravimetric curve (quantification step). After the thermal analysis step, the thermogravimetric curve was further differentiated to determine a component whose weight was reduced in a high-temperature region, and in the quantification step, the weight reduction ratio of the component in the high-temperature region was defined as $\beta$ (mass%), and a content $\gamma$ (mass%) of a non-volatile component (hydrocarbon resin) was determined by $\alpha \times \beta$. The result is shown in the column "Quantification result of content of hydrocarbon resin obtained on the basis of quantification method according to present invention ("PRESENT INVENTION" in Fig. 2) = $\gamma = \alpha \times \beta/100$" in Table 1.

Fig. 2 shows a relationship between a quantification result obtained by the quantification method according to the present invention or the conventional extraction method and an actual content. As can be seen from Table 1 and Fig. 2, the contents of the hydrocarbon resin in all the vulcanized rubbers of the rubber compositions of Formulation No. 1 to Formulation No. 7 determined on the basis of the extraction method are higher than actual contents. On the other hand, the contents of the hydrocarbon resin in all the vulcanized rubbers of the rubber compositions of Formulation No. 1 to Formulation No. 7 derived on the basis of the quantification method according to the present invention are close to actual contents (mass%), and therefore it can be seen that the quantification method according to the present invention is a method for quantifying a component in a rubber composition after vulcanization which is excellent in quantitativeness.

**Claims**

1. A method for quantifying a component in a rubber composition, the method comprising at least:

   an extraction step in which, from the rubber composition, an extraction liquid containing an extract is obtained using an organic solvent;
   a concentration step in which the organic solvent is distilled off from the extraction liquid to obtain a concentrate of the extract;
   a thermal analysis step in which the concentrate is subjected to thermogravimetric analysis to obtain a thermogravimetric curve; and
   a quantification step in which a weight reduction ratio corresponding to a component to be quantified is read from the thermogravimetric curve.

2. The method for quantifying a component in a rubber composition according to claim 1, wherein the component to be quantified is a non-volatile component.

3. The method for quantifying a component in a rubber composition according to claim 2, wherein the non-volatile component is a hydrocarbon resin.

4. The method for quantifying a component in a rubber composition according to any one of claims 1 to 3, wherein

when, in the concentration step, a ratio of extraction amount of the concentrate as determined taking a total amount of the rubber composition as 100 mass% is defined as α (mass%) and

when after the thermal analysis step, a component whose weight is reduced in a high-temperature region is determined by further differentiating the thermogravimetric curve and, in the quantification step, a weight reduction ratio of the component in the high-temperature region is defined as β (mass%),

a content γ (mass%) of the component in the rubber composition is calculated on the basis of the following formula (2) :

$$\gamma = \alpha \times \beta/100 \quad (2).$$

5. The method for quantifying a component in a rubber composition according to any one of claims 1 to 4, wherein the thermal analysis step is performed in an inert gas atmosphere.

6. The method for quantifying a component in a rubber composition according to claim 4 or 5, wherein a differential curve of the thermogravimetric curve corresponding to the component whose weight is reduced in the high-temperature region has a peak top temperature of 250°C or higher.

FIG. 1

FIG. 2

ACTUAL CONTENT (mass%) vs QUANTIFICATION RESULT (mass%)

- ● PRESENT INVENTION
- ◆ EXTRACTION METHOD

Europäisches
Patentamt

European
Patent Office

Office européen
des brevets

# EUROPEAN SEARCH REPORT

**Application Number**

EP 24 18 9039

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Unknown: "GB/T 14837.3-2018: Rubber and rubber products -- Determination of the composition of vulcanizates and uncured compounds by thermogravimetry -- Part 3: Hydrocarbon rubbers, halogenated rubbers and polysiloxane rubbers after extraction", Chinese technical standard, 15 March 2018 (2018-03-15), XP093234037, CN Retrieved from the Internet: URL:https://www.chinesestandard.net/PDF/BOOK.aspx/GBT14837.3-2018 [retrieved on 2024-12-19] * page 5 - page 12 * | 1-6 | INV. G01N33/44 |
| Y | JP 6 665629 B2 (SUMITOMO RUBBER IND) 13 March 2020 (2020-03-13) * paragraphs [0001], [0013], [0017], [0020], [0021], [0030]; claims 1, 4 * | 1-6 | |
| Y | WO 2019/203003 A1 (TORAY ADVANCED FILM CO LTD [JP]) 24 October 2019 (2019-10-24) * paragraphs [0013], [0053] - [0056] * | 1-6 | **TECHNICAL FIELDS SEARCHED (IPC)**<br><br>G01N |
| A | JP 2000 242040 A (CANON KK) 8 September 2000 (2000-09-08) * paragraphs [0157], [0383], [0454], [0527] * | 1-6 | |

The present search report has been drawn up for all claims

| Place of search | Date of completion of the search | Examiner |
|---|---|---|
| The Hague | 19 December 2024 | Martin, Hazel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 506 689 A1**

## ANNEX TO THE EUROPEAN SEARCH REPORT
## ON EUROPEAN PATENT APPLICATION NO.

EP 24 18 9039

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2024

| Patent document cited in search report | | Publication date | Patent family member(s) | | Publication date |
|---|---|---|---|---|---|
| JP 6665629 | B2 | 13-03-2020 | JP | 6665629 B2 | 13-03-2020 |
| | | | JP | 2017181284 A | 05-10-2017 |
| WO 2019203003 | A1 | 24-10-2019 | CN | 111699091 A | 22-09-2020 |
| | | | JP | 7188672 B2 | 13-12-2022 |
| | | | JP | WO2019203003 A1 | 11-03-2021 |
| | | | KR | 20200143358 A | 23-12-2020 |
| | | | TW | 201943564 A | 16-11-2019 |
| | | | WO | 2019203003 A1 | 24-10-2019 |
| JP 2000242040 | A | 08-09-2000 | JP | 4011782 B2 | 21-11-2007 |
| | | | JP | 2000242040 A | 08-09-2000 |

**REFERENCES CITED IN THE DESCRIPTION**

**Patent documents cited in the description**

- JP 6665629 B
- JP 6950494 B